# EUROPEAN PATENT SPECIFICATION

(11) **EP 2 573 173 B1**
(45) Date of publication and mention of the grant of the patent: **11.11.2015**
(21) Application number: 11182725.9
(22) Date of filing: 26.09.2011
(51) Int. Cl.: C12N 9/22, C12N 15/82

(54) **Chimeric nucleases for gene targeting**
Chimäre Nukleasen für Gentargeting
Nucléases chimériques pour ciblage génétique

(43) Date of publication of application: 27.03.2013
(73) Proprietor: Justus-Liebig-Universität Gießen, 35390 Gießen (DE)
(72) Inventor: Pingoud, Alfred, 35582 Wetzlar (DE); Wende, Wolfgang, 35398 Giessen (DE); Mert, Yanik, 35452 Heuchelheim (DE)
(74) Representative: Patentanwälte Olbricht Buchhold Keulertz

(56) References cited:
- US-A1- 2011 201 118
- TING LI ET AL: "TAL nucleases (TALNs): hybrid proteins composed of TAL effectors and FokI DNA-cleavage domain", NUCLEIC ACIDS RESEARCH, OXFORD UNIVERSITY PRESS, SURREY, GB, vol. 39, no. 1, 1 January 2011 (2011-01-01), pages 359-372, XP002659529, ISSN: 0305-1048, DOI: 10.1093/NAR/GKQ704 [retrieved on 2010-08-10]
- C. MUSSOLINO ET AL: "A novel TALE nuclease scaffold enables high genome editing activity in combination with low toxicity", NUCLEIC ACIDS RESEARCH, vol. 39, no. 21, 3 August 2011 (2011-08-03), pages 9283-9293, XP55021128, ISSN: 0305-1048, DOI: 10.1093/nar/gkr597
- EISENSCHMIDT KRISTIN ET AL: "Developing a programmed restriction endonuclease for highly specific DNA cleavage", NUCLEIC ACIDS RESEARCH, OXFORD UNIVERSITY PRESS, SURREY, GB, vol. 33, no. 22, 1 January 2005 (2005-01-01), pages 7039-7047, XP002383230, ISSN: 0305-1048, DOI: 10.1093/NAR/GKI1009
- ALFRED PINGOUD ET AL: "Generation of Novel Nucleases with Extended Specificity by Rational and Combinatorial Strategies", CHEMBIOCHEM, vol. 12, no. 10, 4 July 2011 (2011-07-04), pages 1495-1500, XP55020991, ISSN: 1439-4227, DOI: 10.1002/cbic.201100055
- PINGOUD A ET AL: "Type II restriction endonucleases: structure and mechanism", CMLS CELLULAR AND MOLECULAR LIFE SCIENCES, BIRKHÄUSER-VERLAG, BA, vol. 62, no. 6, 1 March 2005 (2005-03-01), pages 685-707, XP019200964, ISSN: 1420-9071, DOI: 10.1007/S00018-004-4513-1
- I. FONFARA ET AL: "Creating highly specific nucleases by fusion of active restriction endonucleases and catalytically inactive homing endonucleases", NUCLEIC ACIDS RESEARCH, 29 September 2011 (2011-09-29), XP55012639, ISSN: 0305-1048, DOI: 10.1093/nar/gkr788
- B. SCHIERLING ET AL: "A novel zinc-finger nuclease platform with a sequence-specific cleavage module", NUCLEIC ACIDS RESEARCH, 1 December 2011 (2011-12-01), XP55020990, ISSN: 0305-1048, DOI: 10.1093/nar/gkr1112
- MERT YANIK ET AL: "TALE-PvuII Fusion Proteins - Novel Tools for Gene Targeting", PLOS ONE, vol. 8, no. 12, 1 January 2013 (2013-01-01), pages e82539-e82539, XP055118839, ISSN: 1932-6203, DOI: 10.1371/journal.pone.0082539

### SEQUENCE LISTING

<110> Justus-Liebig universitat Giessen
<120> Chimeric Nucleases for Gene Targeting
<130> TM 631
<140> EP
   <141> 2011-09-26
<160> 10
<170> PatentIn version 3.5
<210> 1
   <211> 762
   <212> PRT
   <213> Xanthomonas euvesicatoria
<400> 1
   Val Asp Leu Arg Thr Leu Gly Tyr Ser Gln Gln Gln Gln Glu Lys Ile
   1               5                   10                  15
   Lys Pro Lys Val Arg Ser Thr Val Ala Gln His His Glu Ala Leu Val
               20                  25                  30
   Gly His Gly Phe Thr His Ala His Ile Val Ala Leu Ser Gln His Pro
           35                  40                  45
   Ala Ala Leu Gly Thr Val Ala Val Lys Tyr Gln Asp Met Ile Ala Ala
       50                  55                  60
   Leu Pro Glu Ala Thr His Glu Ala Ile Val Gly Val Gly Lys Gln Trp
   65                  70                  75                  80
   Ser Gly Ala Arg Ala Leu Glu Ala Leu Leu Thr Val Ala Gly Glu Leu
                   85                  90                  95
   Arg Gly Pro Pro Leu Gln Leu Asp Thr Gly Gln Leu Leu Lys Ile Ala
               100                 105                 110
   Lys Arg Gly Gly Val Thr Ala Val Glu Ala Val His Ala Trp Arg Asn
           115                 120                 125
   Ala Leu Thr Gly Ala Pro Leu Asn Leu Thr Pro Glu Gln Val Val Ala
       130                 135                 140
   Ile Ala Ser His Asp Gly Gly Lys Gln Ala Leu Glu Thr Val Gln Arg
   145                 150                 155                 160
   Leu Leu Pro Val Leu Cys Gln Ala His Gly Leu Thr Pro Gln Gln Val
                   165                 170                 175
   Val Ala Ile Ala Ser Asn Gly Gly Gly Lys Gln Ala Leu Glu Thr Val
               180                 185                 190
   Gln Arg Leu Leu Pro Val Leu Cys Gln Ala His Gly Leu Thr Pro Gln
           195                 200                 205
   Gln Val Val Ala Ile Ala Ser Asn Ser Gly Gly Lys Gln Ala Leu Glu
       210                 215                 220
   Thr Val Gln Arg Leu Leu Pro Val Leu Cys Gln Ala His Gly Leu Thr
   225                 230                 235                 240
   Pro Glu Gln Val Val Ala Ile Ala Ser Asn Gly Gly Gly Lys Gln Ala
                   245                 250                 255
   Leu Glu Thr Val Gln Arg Leu Leu Pro Val Leu Cys Gln Ala His Gly
               260                 265                 270
   Leu Thr Pro Glu Gln Val Val Ala Ile Ala Ser Asn Ile Gly Gly Lys
           275                 280                 285
   Gln Ala Leu Glu Thr Val Gln Ala Leu Leu Pro Val Leu Cys Gln Ala
       290                 295                 300
   His Gly Leu Thr Pro Glu Gln Val Val Ala Ile Ala Ser Asn Ile Gly
   305                 310                 315                 320
   Gly Lys Gln Ala Leu Glu Thr Val Gln Ala Leu Leu Pro Val Leu Cys
                   325                 330                 335
   Gln Ala His Gly Leu Thr Pro Glu Gln Val Val Ala Ile Ala Ser Asn
               340                 345                 350
   Ile Gly Gly Lys Gln Ala Leu Glu Thr Val Gln Ala Leu Leu Pro Val
           355                 360                 365
   Leu Cys Gln Ala His Gly Leu Thr Pro Glu Gln Val Val Ala Ile Ala
       370                 375                 380
   Ser His Asp Gly Gly Lys Gln Ala Leu Glu Thr Val Gln Arg Leu Leu
   385                 390                 395                 400
   Pro Val Leu Cys Gln Ala His Gly Leu Thr Pro Glu Gln Val Val Ala
                   405                 410                 415
   Ile Ala Ser His Asp Gly Gly Lys Gln Ala Leu Glu Thr Val Gln Arg
               420                 425                 430
   Leu Leu Pro Val Leu Cys Gln Ala His Gly Leu Thr Pro Gln Gln Val
           435                 440                 445
   Val Ala Ile Ala Ser Asn Gly Gly Gly Lys Gln Ala Leu Glu Thr Val
       450                 455                 460
   Gln Arg Leu Leu Pro Val Leu Cys Gln Ala His Gly Leu Thr Pro Glu
   465 470 475 480
   Gln Val Val Ala Ile Ala Ser Asn Ser Gly Gly Lys Gln Ala Leu Glu
   485 490 495
   Thr Val Gln Ala Leu Leu Pro Val Leu Cys Gln Ala His Gly Leu Thr
   500 505 510
   Pro Glu Gln Val Val Ala Ile Ala Ser Asn Ser Gly Gly Lys Gln Ala
   515 520 525
   Leu Glu Thr Val Gln Arg Leu Leu Pro Val Leu Cys Gln Ala His Gly
   530 535 540
   Leu Thr Pro Glu Gln Val Val Ala Ile Ala Ser His Asp Gly Gly Lys
   545 550 555 560
   Gln Ala Leu Glu Thr Val Gln Arg Leu Leu Pro Val Leu Cys Gln Ala
   565 570 575
   His Gly Leu Thr Pro Glu Gln Val Val Ala Ile Ala Ser His Asp Gly
   580 585 590
   Gly Lys Gln Ala Leu Glu Thr Val Gln Arg Leu Leu Pro Val Leu Cys
   595 600 605
   Gln Ala His Gly Leu Thr Pro Glu Gln Val Val Ala Ile Ala Ser His
   610 615 620
   Asp Gly Gly Lys Gln Ala Leu Glu Thr Val Gln Arg Leu Leu Pro Val
   625 630 635 640
   Leu Cys Gln Ala His Gly Leu Thr Pro Gln Gln Val Val Ala Ile Ala
   645 650 655
   Ser Asn Gly Gly Gly Arg Pro Ala Leu Glu Thr Val Gln Arg Leu Leu
   660 665 670
   Pro Val Leu Cys Gln Ala His Gly Leu Thr Pro Glu Gln Val Val Ala
   675 680 685
   Ile Ala Ser His Asp Gly Gly Lys Gln Ala Leu Glu Thr Val Gln Arg
   690 695 700
   Leu Leu Pro Val Leu Cys Gln Ala His Gly Leu Thr Pro Gln Gln Val
   705 710 715 720
   Val Ala Ile Ala Ser Asn Gly Gly Gly Arg Pro Ala Leu Glu Ser Ile
   725 730 735
   Val Ala Gln Leu Ser Arg Pro Asp Pro Ala Leu Ala Ala Leu Thr Asn
   740 745 750
   Asp His Leu Val Ala Leu Ala Cys Leu Gly
   755 760
<210> 2
   <211> 762
   <212> PRT
   <213> Xanthomonas vesicatoria
<220>
   <221> D524A Pointmutation
   <222> (524)..(524)
   <223> pointmutation D524A
<400> 2
   Val Asp Leu Arg Thr Leu Gly Tyr Ser Gln Gln Gln Gln Glu Lys Ile
   1 5 10 15
   Lys Pro Lys Val Arg Ser Thr Val Ala Gln His His Glu Ala Leu Val
   20 25 30
   Gly His Gly Phe Thr His Ala His Ile Val Ala Leu Ser Gln His Pro
   35 40 45
   Ala Ala Leu Gly Thr Val Ala Val Lys Tyr Gln Asp Met Ile Ala Ala
   50 55 60
   Leu Pro Glu Ala Thr His Glu Ala Ile Val Gly Val Gly Lys Gln Trp
   65 70 75 80
   Ser Gly Ala Arg Ala Leu Glu Ala Leu Leu Thr Val Ala Gly Glu Leu
   85 90 95
   Arg Gly Pro Pro Leu Gln Leu Asp Thr Gly Gln Leu Leu Lys Ile Ala
   100 105 110
   Lys Arg Gly Gly Val Thr Ala Val Glu Ala Val His Ala Trp Arg Asn
   115 120 125
   Ala Leu Thr Gly Ala Pro Leu Asn Leu Thr Pro Glu Gln Val Val Ala
   130 135 140
   Ile Ala Ser Asn Ile Gly Gly Lys Gln Ala Leu Glu Thr Val Gln Ala
   145 150 155 160
   Leu Leu Pro Val Leu Cys Gln Ala His Gly Leu Thr Pro Asp Gln Val
   165 170 175
   Val Ala Ile Ala Ser Asn Gly Gly Gly Lys Gln Ala Leu Glu Thr Val
               180                  185                  190
   Gln Arg Leu Leu Pro Val Leu Cys Gln Ala His Gly Leu Thr Pro Glu
           195                 200                 205
   Gln Val Val Ala Ile Ala Ser Asn Ile Gly Gly Lys Gln Ala Leu Glu
       210                 215                 220
   Thr Val Gln Arg Leu Leu Pro Val Leu Cys Gln Ala His Gly Leu Thr
   225                 230                 235                 240
   Pro Glu Gln Val Val Ala Ile Ala Ser Asn Ile Gly Gly Lys Gln Ala
                   245                 250                 255
   Leu Glu Thr Val Gln Arg Leu Leu Pro Val Leu Cys Gln Ala His Gly
               260                 265                 270
   Leu Thr Pro Glu Gln Val Val Ala Ile Ala Ser Asn Gly Gly Gly Lys
           275                 280                 285
   Gln Ala Leu Glu Thr Val Gln Arg Leu Leu Pro Val Leu Cys Gln Ala
       290                 295                 300
   His Gly Leu Thr Pro Glu Gln Val Val Ala Ile Ala Ser Asn Gly Gly
   305                 310                 315                 320
   Gly Lys Gln Ala Leu Glu Thr Val Gln Arg Leu Leu Pro Val Leu Cys
                   325                 330                 335
   Gln Ala His Gly Leu Ile Pro Gln Gln Val Val Ala Ile Ala Ser Asn
               340                 345                 350
   Ile Gly Gly Lys Gln Ala Leu Glu Thr Val Gln Arg Leu Leu Pro Val
           355                 360                 365
   Leu Cys Gln Ala His Gly Leu Thr Pro Gln Gln Val Val Ala Ile Ala
       370                 375                 380
   Ser Asn Ser Gly Gly Lys Gln Ala Leu Glu Thr Val Gln Arg Leu Leu
   385                 390                 395                 400
   Pro Val Leu Cys Gln Ala His Gly Leu Thr Pro Glu Gln Val Val Ala
                   405                 410                 415
   Ile Ala Ser Asn Gly Gly Gly Lys Gln Ala Leu Glu Thr Val Gln Arg
               420                 425                 430
   Leu Leu Pro Val Leu Cys Gln Ala His Gly Leu Thr Pro Gln Gln Val
           435                 440                 445
   Val Ala Ile Ala Ser Asn Ile Gly Gly Lys Gln Ala Leu Glu Thr Val
   450 455 460
   Gln Arg Leu Leu Pro Val Leu Cys Gln Asp His Gly Leu Thr Pro Gln
   465 470 475 480
   Gln Val Val Ala Ile Ala Ser Asn Ser Gly Gly Lys Gln Ala Leu Glu
   485 490 495
   Thr Val Gln Arg Leu Leu Pro Val Leu Cys Gln Ala His Gly Leu Thr
   500 505 510
   Pro Gln Gln Val Val Ala Ile Ala Ser Asn Gly Gly Gly Lys Gln Ala
   515 520 525
   Leu Glu Thr Val Gln Arg Leu Leu Pro Val Leu Cys Gln Ala His Gly
   530 535 540
   Leu Thr Pro Gln Gln Val Val Ala Ile Ala Ser His Asp Gly Gly Lys
   545 550 555 560
   Gln Ala Leu Glu Thr Val Gln Arg Leu Leu Pro Val Leu Cys Gln Ala
   565 570 575
   His Gly Leu Thr Pro Gln Gln Val Val Ala Ile Ala Ser His Asp Gly
   580 585 590
   Gly Lys Gln Ala Leu Glu Thr Val Gln Arg Leu Leu Pro Val Leu Cys
   595 600 605
   Gln Ala His Gly Leu Thr Pro Gln Gln Val Val Ala Ile Ala Ser Asn
   610 615 620
   Ser Gly Gly Lys Gln Ala Leu Glu Thr Val Gln Arg Leu Leu Pro Val
   625 630 635 640
   Leu Cys Gln Ala His Gly Leu Thr Pro Gln Gln Val Val Ala Ile Ala
   645 650 655
   Ser His Asp Gly Gly Lys Gln Ala Leu Glu Thr Val Gln Arg Leu Leu
   660 665 670
   Pro Val Leu Cys Gln Ala His Gly Leu Thr Pro Gln Gln Val Val Ala
   675 680 685
   Ile Ala Ser Asn Gly Gly Gly Lys Gln Ala Leu Glu Thr Val Gln Arg
   690 695 700
   Leu Leu Pro Val Leu Cys Gln Ala His Gly Leu Thr Pro Gln Gln Val
   705 710 715 720
   Val Ala Ile Ala Ser Asn Gly Gly Gly Arg Pro Ala Leu Glu Ser Ile
   725 730 735
   Val Ala Gln Leu Ser Arg Pro Asp Pro Ala Leu Ala Ala Leu Thr Asn
   740 745 750
   Asp His Leu Val Ala Leu Ala Cys Leu Gly
   755 760
<210> 3
   <211> 156
   <212> PRT
   <213> Proteus hauseri
<220>
   <221> Pointmutation T46G
   <222> (46)..(46)
   <223> Pointmutation T46G im Vergleich zum Wildtyp
<400> 3
   Ser His Pro Asp Leu Asn Lys Leu Leu Glu Leu Trp Pro His Ile Gln
   1 5 10 15
   Glu Tyr Gln Asp Leu Ala Leu Lys His Gly Ile Asn Asp Ile Phe Gln
   20 25 30
   Asp Asn Gly Gly Lys Leu Leu Gln Val Leu Leu Ile Gly Gly Leu Thr
   35 40 45
   Val Leu Pro Gly Arg Glu Gly Asn Asp Ala Val Asp Asn Ala Gly Gln
   50 55 60
   Glu Tyr Glu Leu Lys Ser Ile Asn Ile Asp Leu Thr Lys Gly Phe Ser
   65 70 75 80
   Thr His His His Met Asn Pro Val Ile Ile Ala Lys Tyr Arg Gln Val
   85 90 95
   Pro Trp Ile Phe Ala Ile Tyr Arg Gly Ile Ala Ile Glu Ala Ile Tyr
   100 105 110
   Arg Leu Glu Pro Lys Asp Leu Glu Phe Tyr Tyr Asp Lys Trp Glu Arg
   115 120 125
   Lys Trp Tyr Ser Asp Gly His Lys Asp Ile Asn Asn Pro Lys Ile Pro
   130 135 140
   Val Lys Tyr Val Met Glu His Gly Thr Lys Ile Tyr
   145 150 155
<210> 4
   <211> 15
   <212> PRT
   <213> articial sequence
<400> 4
   Ser Ser Val Ile Pro Asn Arg Gly Val Thr Lys Gln Leu Val Lys
   1               5                   10                  15
<210> 5
   <211> 992
   <212> PRT
   <213> artificial sequence
<220>
   <223> fusion protein
<220>
   <221> StrepII
   <222> (5)..(12)
<220>
   <221> AvrBs4
   <222> (16)..(812)
   <223> AvrBs4 (153-914)
<220>
   <221> Linker
   <222> (813)..(827)
   <223> Linker
<220>
   <221> PvuII
   <222> (828)..(986)
   <223> PvuII (2-157; T46G)
<220>
   <221> 6His
   <222> (987)..(992)
<400> 5
   Met His Thr Ser Trp Ser His Pro Gln Phe Glu Lys Ser Thr Gly Val
   1               5                   10                  15
   Asp Leu Arg Thr Leu Gly Tyr Ser Gln Gln Gln Gln Glu Lys Ile Lys
               20                  25                  30
   Pro Lys Val Arg Ser Thr Val Ala Gln His His Glu Ala Leu Val Gly
           35                  40                  45
   His Gly Phe Thr His Ala His Ile Val Ala Leu Ser Gln His Pro Ala
       50                  55                  60
   Ala Leu Gly Thr Val Ala Val Lys Tyr Gln Asp Met Ile Ala Ala Leu
   65                  70                  75                  80
   Pro Glu Ala Thr His Glu Ala Ile Val Gly Val Gly Lys Gln Trp Ser
                   85                  90                  95
   Gly Ala Arg Ala Leu Glu Ala Leu Leu Thr Val Ala Gly Glu Leu Arg
               100                 105                 110
   Gly Pro Pro Leu Gln Leu Asp Thr Gly Gln Leu Leu Lys Ile Ala Lys
           115                 120                 125
   Arg Gly Gly Val Thr Ala Val Glu Ala Val His Ala Trp Arg Asn Ala
       130                 135                 140
   Leu Thr Gly Ala Pro Leu Asn Leu Thr Pro Glu Gln Val Val Ala Ile
   145                 150                 155                 160
   Ala Ser Asn Ile Gly Gly Lys Gln Ala Leu Glu Thr Val Gln Ala Leu
                   165                 170                 175
   Leu Pro Val Leu Cys Gln Ala His Gly Leu Thr Pro Asp Gln Val Val
               180                 185                 190
   Ala Ile Ala Ser Asn Gly Gly Gly Lys Gln Ala Leu Glu Thr Val Gln
           195                 200                 205
   Arg Leu Leu Pro Val Leu Cys Gln Ala His Gly Leu Thr Pro Glu Gln
       210                 215                 220
   Val Val Ala Ile Ala Ser Asn Ile Gly Gly Lys Gln Ala Leu Glu Thr
   225                 230                 235                 240
   Val Gln Arg Leu Leu Pro Val Leu Cys Gln Ala His Gly Leu Thr Pro
                   245                 250                 255
   Glu Gln Val Val Ala Ile Ala Ser Asn Ile Gly Gly Lys Gln Ala Leu
               260                 265                 270
   Glu Thr Val Gln Arg Leu Leu Pro Val Leu Cys Gln Ala His Gly Leu
           275                 280                 285
   Thr Pro Glu Gln Val Val Ala Ile Ala Ser Asn Gly Gly Gly Lys Gln
       290                 295                 300
   Ala Leu Glu Thr Val Gln Arg Leu Leu Pro Val Leu Cys Gln Ala His
   305                 310                 315                 320
   Gly Leu Thr Pro Glu Gln Val Val Ala Ile Ala Ser Asn Gly Gly Gly
                   325                 330                 335
   Lys Gln Ala Leu Glu Thr Val Gln Arg Leu Leu Pro Val Leu Cys Gln
               340                 345                 350
   Ala His Gly Leu Ile Pro Gln Gln Val Val Ala Ile Ala Ser Asn Ile
           355                 360                 365
   Gly Gly Lys Gln Ala Leu Glu Thr Val Gln Arg Leu Leu Pro Val Leu
       370                 375                 380
   Cys Gln Ala His Gly Leu Thr Pro Gln Gln Val Val Ala Ile Ala Ser
   385                 390                 395                 400
   Asn Ser Gly Gly Lys Gln Ala Leu Glu Thr Val Gln Arg Leu Leu Pro
                   405                 410                 415
   Val Leu Cys Gln Ala His Gly Leu Thr Pro Glu Gln Val Val Ala Ile
               420                 425                 430
   Ala Ser Asn Gly Gly Gly Lys Gln Ala Leu Glu Thr Val Gln Arg Leu
           435                 440                 445
   Leu Pro Val Leu Cys Gln Ala His Gly Leu Thr Pro Gln Gln Val Val
       450                 455                 460
   Ala Ile Ala Ser Asn Ile Gly Gly Lys Gln Ala Leu Glu Thr Val Gln
   465                 470                 475                 480
   Arg Leu Leu Pro Val Leu Cys Gln Asp His Gly Leu Thr Pro Gln Gln
                   485                 490                 495
   Val Val Ala Ile Ala Ser Asn Ser Gly Gly Lys Gln Ala Leu Glu Thr
               500                 505                 510
   Val Gln Arg Leu Leu Pro Val Leu Cys Gln Ala His Gly Leu Thr Pro
           515                 520                 525
   Gln Gln Val Val Ala Ile Ala Ser Asn Gly Gly Gly Lys Gln Ala Leu
       530                 535                 540
   Glu Thr Val Gln Arg Leu Leu Pro Val Leu Cys Gln Ala His Gly Leu
   545                 550                 555                 560
   Thr Pro Gln Gln Val Val Ala Ile Ala Ser His Asp Gly Gly Lys Gln
                   565                 570                 575
   Ala Leu Glu Thr Val Gln Arg Leu Leu Pro Val Leu Cys Gln Ala His
               580                 585                 590
   Gly Leu Thr Pro Gln Gln Val Val Ala Ile Ala Ser His Asp Gly Gly
           595                 600                 605
   Lys Gln Ala Leu Glu Thr Val Gln Arg Leu Leu Pro Val Leu Cys Gln
       610                 615                 620
   Ala His Gly Leu Thr Pro Gln Gln Val Val Ala Ile Ala Ser Asn Ser
   625                 630                 635                 640
   Gly Gly Lys Gln Ala Leu Glu Thr Val Gln Arg Leu Leu Pro Val Leu
                   645                 650                 655
   Cys Gln Ala His Gly Leu Thr Pro Gln Gln Val Val Ala Ile Ala Ser
               660                 665                 670
   His Asp Gly Gly Lys Gln Ala Leu Glu Thr Val Gln Arg Leu Leu Pro
           675                 680                 685
   Val Leu Cys Gln Ala His Gly Leu Thr Pro Gln Gln Val Val Ala Ile
       690                 695                 700
   Ala Ser Asn Gly Gly Gly Lys Gln Ala Leu Glu Thr Val Gln Arg Leu
   705                 710                 715                 720
   Leu Pro Val Leu Cys Gln Ala His Gly Leu Thr Pro Gln Gln Val Val
                   725                 730                 735
   Ala Ile Ala Ser Asn Gly Gly Gly Arg Pro Ala Leu Glu Ser Ile Val
               740                 745                 750
   Ala Gln Leu Ser Arg Pro Asp Pro Ala Leu Ala Ala Leu Thr Asn Asp
           755                 760                 765
   His Leu Val Ala Leu Ala Cys Leu Gly Gly Arg Pro Ala Leu Asp Ala
       770                 775                 780
   Val Lys Lys Gly Leu Pro His Ala Pro Ala Leu Ile Lys Arg Thr Asn
   785                 790                 795                 800
   Arg Arg Ile Pro Glu Arg Thr Ser His Arg Val Ala Ser Ser Val Ile
                   805                 810                 815
   Pro Asn Arg Gly Val Thr Lys Gln Leu Val Lys Gly Thr His Pro Asp
               820                 825                 830
   Leu Asn Lys Leu Leu Glu Leu Trp Pro His Ile Gln Glu Tyr Gln Asp
           835                 840                 845
   Leu Ala Leu Lys His Gly Ile Asn Asp Ile Phe Gln Asp Asn Gly Gly
       850                 855                 860
   Lys Leu Leu Gln Val Leu Leu Ile Gly Gly Leu Thr Val Leu Pro Gly
   865                 870                 875                 880
   Arg Glu Gly Asn Asp Ala Val Asp Asn Ala Gly Gln Glu Tyr Glu Leu
                   885                 890                 895
   Lys Ser Ile Asn Ile Asp Leu Thr Lys Gly Phe Ser Thr His His His
               900                 905                 910
   Met Asn Pro Val Ile Ile Ala Lys Tyr Arg Gln Val Pro Trp Ile Phe
           915                 920                 925
   Ala Ile Tyr Arg Gly Ile Ala Ile Glu Ala Ile Tyr Arg Leu Glu Pro
       930                 935                 940
   Lys Asp Leu Glu Phe Tyr Tyr Asp Lys Trp Glu Arg Lys Trp Tyr Ser
   945                 950                 955                 960
   Asp Gly His Lys Asp Ile Asn Asn Pro Lys Ile Pro Val Lys Tyr Val
                   965                 970                 975
   Met Glu His Gly Thr Lys Ile Tyr Gly Ser His His His His His His
               980                 985                 990
<210> 6
   <211> 1117
   <212> PRT
   <213> artificial sequence
<220>
   <223> fusion protein
<220>
   <221> StrepII
   <222> (5)..(12)
<220>
   <221> AvrBs3
   <222> (16)..(777)
   <223> AvrBs3 (153-914)
<220>
   <221> Linker
   <222> (778)..(792)
   <223> Linker
<220>
   <221> PvuII (1)
   <222> (778)..(792)
   <223> 1. PvuII
<220>
   <221> PvuII (2)
   <222> (793)..(1111)
   <223> 2. PvuII
<220>
   <221> 6His
   <222> (1112)..(1117)
<400> 6
   Met His Thr Ser Trp Ser His Pro Gln Phe Glu Lys Ser Thr Gly Val
   1 5 10 15
   Asp Leu Arg Thr Leu Gly Tyr Ser Gln Gln Gln Gln Glu Lys Ile Lys
   20 25 30
   Pro Lys Val Arg Ser Thr Val Ala Gln His His Glu Ala Leu Val Gly
   35 40 45
   His Gly Phe Thr His Ala His Ile Val Ala Leu Ser Gln His Pro Ala
       50                  55                  60
   Ala Leu Gly Thr Val Ala Val Lys Tyr Gln Asp Met Ile Ala Ala Leu
   65                  70                  75                  80
   Pro Glu Ala Thr His Glu Ala Ile Val Gly Val Gly Lys Gln Trp Ser
                   85                  90                  95
   Gly Ala Arg Ala Leu Glu Ala Leu Leu Thr Val Ala Gly Glu Leu Arg
               100                 105                 110
   Gly Pro Pro Leu Gln Leu Asp Thr Gly Gln Leu Leu Lys Ile Ala Lys
           115                 120                 125
   Arg Gly Gly Val Thr Ala Val Glu Ala Val His Ala Trp Arg Asn Ala
       130                 135                 140
   Leu Thr Gly Ala Pro Leu Asn Leu Thr Pro Glu Gln Val Val Ala Ile
   145                 150                 155                 160
   Ala Ser His Asp Gly Gly Lys Gln Ala Leu Glu Thr Val Gln Arg Leu
                   165                 170                 175
   Leu Pro Val Leu Cys Gln Ala His Gly Leu Thr Pro Gln Gln Val Val
               180                 185                 190
   Ala Ile Ala Ser Asn Gly Gly Gly Lys Gln Ala Leu Glu Thr Val Gln
           195                 200                 205
   Arg Leu Leu Pro Val Leu Cys Gln Ala His Gly Leu Thr Pro Gln Gln
       210                 215                 220
   Val Val Ala Ile Ala Ser Asn Ser Gly Gly Lys Gln Ala Leu Glu Thr
   225                 230                 235                 240
   Val Gln Arg Leu Leu Pro Val Leu Cys Gln Ala His Gly Leu Thr Pro
                   245                 250                 255
   Glu Gln Val Val Ala Ile Ala Ser Asn Gly Gly Gly Lys Gln Ala Leu
               260                 265                 270
   Glu Thr Val Gln Arg Leu Leu Pro Val Leu Cys Gln Ala His Gly Leu
           275                 280                 285
   Thr Pro Glu Gln Val Val Ala Ile Ala Ser Asn Ile Gly Gly Lys Gln
       290                 295                 300
   Ala Leu Glu Thr Val Gln Ala Leu Leu Pro Val Leu Cys Gln Ala His
   305                 310                 315                 320
   Gly Leu Thr Pro Glu Gln Val Val Ala Ile Ala Ser Asn Ile Gly Gly
   325 330 335
   Lys Gln Ala Leu Glu Thr Val Gln Ala Leu Leu Pro Val Leu Cys Gln
   340 345 350
   Ala His Gly Leu Thr Pro Glu Gln Val Val Ala Ile Ala Ser Asn Ile
   355 360 365
   Gly Gly Lys Gln Ala Leu Glu Thr Val Gln Ala Leu Leu Pro Val Leu
   370 375 380
   Cys Gln Ala His Gly Leu Thr Pro Glu Gln Val Val Ala Ile Ala Ser
   385 390 395 400
   His Asp Gly Gly Lys Gln Ala Leu Glu Thr Val Gln Arg Leu Leu Pro
   405 410 415
   Val Leu Cys Gln Ala His Gly Leu Thr Pro Glu Gln Val Val Ala Ile
   420 425 430
   Ala Ser His Asp Gly Gly Lys Gln Ala Leu Glu Thr Val Gln Arg Leu
   435 440 445
   Leu Pro Val Leu Cys Gln Ala His Gly Leu Thr Pro Gln Gln Val Val
   450 455 460
   Ala Ile Ala Ser Asn Gly Gly Gly Lys Gln Ala Leu Glu Thr Val Gln
   465 470 475 480
   Arg Leu Leu Pro Val Leu Cys Gln Ala His Gly Leu Thr Pro Glu Gln
   485 490 495
   Val Val Ala Ile Ala Ser Asn Ser Gly Gly Lys Gln Ala Leu Glu Thr
   500 505 510
   Val Gln Ala Leu Leu Pro Val Leu Cys Gln Ala His Gly Leu Thr Pro
   515 520 525
   Glu Gln Val Val Ala Ile Ala Ser Asn Ser Gly Gly Lys Gln Ala Leu
   530 535 540
   Glu Thr Val Gln Arg Leu Leu Pro Val Leu Cys Gln Ala His Gly Leu
   545 550 555 560
   Thr Pro Glu Gln Val Val Ala Ile Ala Ser His Asp Gly Gly Lys Gln
   565 570 575
   Ala Leu Glu Thr Val Gln Arg Leu Leu Pro Val Leu Cys Gln Ala His
   580 585 590
   Gly Leu Thr Pro Glu Gln Val Val Ala Ile Ala Ser His Asp Gly Gly
   595 600 605
   Lys Gln Ala Leu Glu Thr Val Gln Arg Leu Leu Pro Val Leu Cys Gln
   610 615 620
   Ala His Gly Leu Thr Pro Glu Gln Val Val Ala Ile Ala Ser His Asp
   625 630 635 640
   Gly Gly Lys Gln Ala Leu Glu Thr Val Gln Arg Leu Leu Pro Val Leu
   645 650 655
   Cys Gln Ala His Gly Leu Thr Pro Gln Gln Val Val Ala Ile Ala Ser
   660 665 670
   Asn Gly Gly Gly Arg Pro Ala Leu Glu Thr Val Gln Arg Leu Leu Pro
   675 680 685
   Val Leu Cys Gln Ala His Gly Leu Thr Pro Glu Gln Val Val Ala Ile
   690 695 700
   Ala Ser His Asp Gly Gly Lys Gln Ala Leu Glu Thr Val Gln Arg Leu
   705 710 715 720
   Leu Pro Val Leu Cys Gln Ala His Gly Leu Thr Pro Gln Gln Val Val
   725 730 735
   Ala Ile Ala Ser Asn Gly Gly Gly Arg Pro Ala Leu Glu Ser Ile Val
   740 745 750
   Ala Gln Leu Ser Arg Pro Asp Pro Ala Leu Ala Ala Leu Thr Asn Asp
   755 760 765
   His Leu Val Ala Leu Ala Cys Leu Gly Ser Ser Val Ile Pro Asn Arg
   770 775 780
   Gly Val Thr Lys Gln Leu Val Lys Gly Thr His Pro Asp Leu Asn Lys
   785 790 795 800
   Leu Leu Glu Leu Trp Pro His Ile Gln Glu Tyr Gln Asp Leu Ala Leu
   805 810 815
   Lys His Gly Ile Asn Asp Ile Phe Gln Asp Asn Gly Gly Lys Leu Leu
   820 825 830
   Gln Val Leu Leu Ile Gly Gly Leu Thr Val Leu Pro Gly Arg Glu Gly
   835 840 845
   Asn Asp Ala Val Asp Asn Ala Gly Gln Glu Tyr Glu Leu Lys Ser Ile
   850 855 860
   Asn Ile Asp Leu Thr Lys Gly Phe Ser Thr His His His Met Asn Pro
   865                 870                 875                 880
   Val Ile Ile Ala Lys Tyr Arg Gln Val Pro Trp Ile Phe Ala Ile Tyr
                   885                 890                 895
   Arg Gly Ile Ala Ile Glu Ala Ile Tyr Arg Leu Glu Pro Lys Asp Leu
               900                 905                 910
   Glu Phe Tyr Tyr Asp Lys Trp Glu Arg Lys Trp Tyr Ser Asp Gly His
           915                 920                 925
   Lys Asp Ile Asn Asn Pro Lys Ile Pro Val Lys Tyr Val Met Glu His
       930                 935                 940
   Gly Thr Lys Ile Tyr Gly Ser Gly Gly Ser His Pro Asp Leu Asn Lys
   945                 950                 955                 960
   Leu Leu Glu Leu Trp Pro His Ile Gln Glu Tyr Gln Asp Leu Ala Leu
                   965                 970                 975
   Lys His Gly Ile Asn Asp Ile Phe Gln Asp Asn Gly Gly Lys Leu Leu
               980                 985                 990
   Gln Val Leu Leu Ile Gly Gly Leu  Thr Val Leu Pro Gly  Arg Glu Gly
           995                 1000                 1005
   Asn Asp  Ala Val Asp Asn Ala  Gly Gln Glu Tyr Glu  Leu Lys Ser
       1010                 1015                 1020
   Ile Asn  Ile Asp Leu Thr Lys  Gly Phe Ser Thr His  His His Met
       1025                 1030                 1035
   Asn Pro  Val Ile Ile Ala Lys  Tyr Arg Gln Val Pro  Trp Ile Phe
       1040                 1045                 1050
   Ala Ile  Tyr Arg Gly Ile Ala  Ile Glu Ala Ile Tyr  Arg Leu Glu
       1055                 1060                 1065
   Pro Lys  Asp Leu Glu Phe Tyr  Tyr Asp Lys Trp Glu  Arg Lys Trp
       1070                 1075                 1080
   Tyr Ser  Asp Gly His Lys Asp  Ile Asn Asn Pro Lys  Ile Pro Val
       1085                 1090                 1095
   Lys Tyr  Val Met Glu His Gly  Thr Lys Ile Tyr Gly  Ser His His
       1100                 1105                 1110
   His His  His His
       1115
<210> 7
   <211> 60
   <212> DNA
   <213> DNA target site
<400> 7
   tatataaacc taaccatccg gatcccagct gtctagagga tggttaggtt tatatacatg 60
<210> 8
   <211> 43
   <212> DNA
   <213> primer sequence
<400> 8
   agttcgagaa gtcgaccggg gttgatctta gaacattggg ata 43
<210> 9
   <211> 34
   <212> DNA
   <213> primer sequence
<220>
   <221> TALE reverse 1
   <222> (1)..(34)
   <223> TALE reverse 1: for long variant TALE-PvuII
<400> 9
   gataacggag ctcgctactc tatggctggt gcgc 34
<210> 10
   <211> 34
   <212> DNA
   <213> primer sequence
<220>
   <221> TALE reverse 2
   <222> (1)..(34)
   <223> TALE reverse 2: for short variant TALE-scPvuII
<400> 10
   ggataacgga gctccctagg catgctaaag caac 34

## Description

The invention relates to a chimeric nuclease comprising a module with at least a DNA binding domain from TALE protein AvrBs3 and/or a DNA binding domain from TALE protein AvrBs4, a linker composed of at least five amino acid residues, and a module comprising at least a cleavage domain of type IIP restriction endonuclease Pvull. The invention further relates to a vector encoding said chimeric nuclease, a method for manufacturing said chimeric nuclease and a non-human organism comprising said chimeric nuclease or vector.

### Background of the invention

Gene targeting, i.e. targeted gene knockout or gene replacement, requires nucleases that recognize unique sequences and cleave DNA highly specifically, and thereby initiate non-homologous end joining which can inactivate genes, or, when a template DNA is present, leads to a replacement of a genetic element by homologous recombination. The specificity of these highly specific nucleases must be such that only a single double-strand DNA (dsDNA) cut is made in a complex genome. A widely used approach is to use fusion proteins consisting of zinc fingers (ZFs) or transcription activator-like effector (TALE) proteins as programmable DNA binding modules with the non-specific DNA cleavage domain of the restriction endonuclease (synonymically also designated as restriction enzyme herein below) Fokl.

Chimeric nuclease constructs comprising a TALE DNA-binding domain and a endonuclease Fokl domain have been shown (TING Li et al., TAL nucleases (TALNs): hybrid proteins composed of TAL effectors and Fokl DNA-cleavage domain. Nucl. Acids Res. January 2011, Vol. 39, No. 1, pages 359-372; C. Mussolino et al., A novel TALE nuclease scaffold enables high genome editing activity in combination with low toxicity. Nucl. Acids Res. August 2011, Vol. 39, No. 21, pages 9283-9293.).

In addition, US 2011/201118 A1 proposes fusion polypeptides comprising a TAL effector (TALE) binding domain and cleavage domain for targeted cleavage of cellular cromatin in a region of interest and/or homologous recombination at a predetermined site in cells. The cleavage domain can be a Type IIS restriction endonuclease, in particular Fokl.

Recent systematic analyses have shown that the zinc finger nucleases (ZFNs) occasionally cleave non-addressed sequences ("off-target sites"). Furthermore, it is believed that the DNA cleavage domain of the restriction endonuclease Fokl has principal limitations.

Conjugates of single chain Pvull coupled to a triple-helix forming oligonucleotide (TFO) via a bifunctional cross-linker have been proposed (K. Eisenschmidt et al., Developing a programmed restriction endonuclease for highly specific DNA cleavage. Nucl. Acids Res. January 2005, Vol. 33, No. 22, pages 7039-7047.) This artificial nuclease has a bipartite recognition sequence. For this construct a bifunctional cross-linker, a succinimide ester, was used for coupling of both domains. The *in vivo* applicability of such a construct is however impaired due to the requirement of a preincubation step in the absence of Mg²⁺.

The goal of gene targeting is targeted gene knockout or gene replacement. One of the most promising and simple strategies is based on homologous recombination, in which the DNA sequence of interest is supplied *in trans* to stimulate site-specific recombination. Ideally, the gene of interest is deleted, modified or replaced in its natural context while leaving the rest of the genome untouched. A major obstacle for the homologous recombination-driven approach is the low efficiency of natural recombination between an introduced DNA and the homologous chromosomal target. Remarkably, it was discovered that making a specific double-strand break in the target sequence increases the frequency of homologous recombination by several orders of magnitude. To facilitate such double-strand break-induced recombination, zinc finger nucleases, engineered homing endonucleases and chimeric restriction endonucleases have been created and used in experimental systems. Chimeric nucleases therefore hold great potential for gene targeting as they allow to cleave DNA at a chosen target sequence.

### Technical problem

However, known chimeric nucleases (synonymically also designated as fusion proteins herein below) also cleave prolifically at off-target sites, resulting in unacceptable toxicity, and are not programmable. Toxicity due to off-target cleavage is a major issue in all gene targeting efforts. The off-target cleavage activity of the known chimeric nucleases is related to missing suppression of restriction enzyme cleavage at restriction enzyme cleavage sites at the DNA that are not addressed by the highly specific DNA binding module of the fusion protein. Thus, there is a need for chimeric nucleases that do not cleave at off-target sites in the DNA but highly specifically address bipartite or tripartite DNA target sites resulting in significantly reduced toxicity in gene targeting efforts suitable for *in vivo* application.

### Solution of the problem

In one aspect, the inventin provides a chimeric nuclease which comprises the following components:
- a module comprising at least a DNA binding domain from TALE protein AvrBs3 (SEQ ID NO: 1) and/or a DNA binding domain from TALE protein AvrBs4 (SEQ I D NO: 2),
- a linker composed of at least five amino acid residues, and
- a module comprising at least a cleavage domain of a type IIP restriction endonuclease Pvull either having an amino acid sequence according to SEQ ID NO: 3 or having an amino acid sequence according to SEQ ID NO: 3 with one of following modifications: amino acid substitutions D5K, L12E, P14G, H15D, Q17E, K25D, K25E, N62D, K78E, H83A, Y94F, K155E, or K174E, respectively, or combinations thereof, or amino acid deletions delta 1-15 or delta 1-25 (positions referring to wildtype Pvull),
wherein said chimeric nuclease functionally interacts only with DNA that comprises both a DNA recognition site for a DNA binding domain derived from a TALE protein and a DNA recognition site for a cleavage domain derived from a restriction endonuclease, and wherein said cleavage domain cleaves the DNA upon binding of the chimeric nuclease to the DNA.

The aforementioned technical problem is thus solved by creation of said chimeric nucleases consisting of a restriction enzyme cleavage module and transcription activator-like effector (TALE) protein(s) as DNA binding module(s) that in contrast to the prior art exhibit increased preference and specificity to bipartite or tripartite target sites without cleaving restriction enzyme off-target sites and that are programmable.

Moreover, surprisingly it is found in the invention that amino acid substitutions introduced into a restriction enzyme that forms the cleavage module of the chimeric nuclease, namely Pvull that cleaves double-stranded DNA at the sequence CAG↓CTG, suppress cleavage by the restriction enzyme at restriction enzyme cleavage sites that are not addressed by the highly specific TALE protein(s) as DNA binding module(s) of the fusion protein.

Transcription activator-like effector (TALE) proteins combine two advantages: they are programmable and recognize their DNA target sequence highly specific. TAL effectors (TALEs) are proteins secreted by Xanthomonas bacteria via their type III secretion system when they infect various plant species. These proteins can bind promoter sequences in the host and activate the expression of host genes that aid bacterial infection. These proteins are interesting both for their role in disease, for example of important crop species, and the relative ease of retargeting them to bind new DNA sequences. Similar proteins are found in the pathogenic bacteria *Ralstonia solanacearum.* The most distinctive characteristic of TALE proteins is a central repeat domain containing between 1.5 and 33.5 repeats that are usually 34 residues in length (the C-terminal repeat is generally shorter and referred to as a "half repeat").

A typical repeat sequence is LTPEQVVAIASHDGGKQALETVQRLLPVLCQAHG, but the residues at the 12th and 13th positions are hypervariable (these two amino acids are also known as the repeat variable diresidue or RVD). It has been shown that there is a simple relationship between the identity of these two residues in sequential repeats and sequential DNA bases in the TAL effector's target site. There appears to be a one-to-one correspondence between the identity of two critical amino acids in each repeat and each DNA base in the target sequence.

TALE proteins for the DNA binding domain of the chimeric nucleases according to the invention herein are selected from the Avirulence protein AvrBs3 (SEQ ID No. 1) and/or AvrBs4 (SEQ ID No. 2), for example of *Xanthomonas euvesicatoria* or *Xanthomonas vesicatoria,* respectively.

Restriction endonucleases (also called restriction enzymes) as referred to herein in accordance with the present invention are capable of recognizing and cleaving a DNA molecule at a specific DNA cleavage site between predefined nucleotides. In contrast, some endonucleases such as for example Fokl comprise a cleavage domain that cleaves the DNA unspecifically at a certain position regardless of the nucleotides present at this position. Therefore, the specific DNA cleavage site and the DNA recognition site of the restriction endonuclease are identical. Moreover, the cleavage domain of the chimeric nuclease is derived from a restriction endonuclease with reduced DNA binding and/or reduced catalytic activity when compared to the wildtype restriction endonuclease.

According to the knowledge that restriction endonucleases, particularly type II restriction endonucleases, bind as a homodimer to DNA regularly, the chimeric nucleases as referred to herein are related to homodimerization of two restriction endonucleases subunits. Preferably, in accordance with the present invention the cleavage modules referred to herein have a reduced capability of forming homodimers in the absence of the DNA recognition site, thereby preventing unspecific DNA binding. Therefore, a functional homodimer is only formed upon recruitment of chimeric nucleases monomers to the specific DNA recognition sites.

The restriction endonuclease of the cleavage module of the chimeric nuclease is a type IIP restriction endonuclease. The palindromic DNA recognition sites of these restriction endonucleases consist of at least four or up to eight contiguous nucleotides. Type IIP restriction endonucleases cleave the DNA within the recognition site which occurs rather frequently in the genome, or immediately adjacent thereto, and have no or a reduced star activity.

The restriction endonuclease according to the invention is a genetically modified variant of Pvull as defined above. The introduction of said amino acid substitutions or deletions results in cleavage of the DNA by the chimeric TALE-Pvull fusion protein only at sequences that are addressed by the TALE protein (SEQ ID No. 7).

The T46G variant Pvull amino acid sequence referred to herein is according to SEQ ID No. 3 or is a modified variant thereof which differs from SEQ ID No. 3 by at least one of above defined amino acid substitution and/or deletion.

The DNA binding domain and the cleavage domain according to the present invention are separated within the chimeric nuclease by a linker allowing interaction with the addressed DNA binding site and cleavage at the DNA restriction site. The flexible linker structures as referred to the invention herein consist of five to twenty amino acids. More preferably, the linker comprises at least six or up to ten or more amino acids. In one embodiment, said linker has an amino acid sequence as shown in SEQ ID No. 4 (SSVIPNRGVTKQLVK).

According to the present invention, the DNA binding domain from said TALE protein and the cleavage domain from said restriction endonuclease are fused by an amino acid linker to generate a specific nuclease. Said TALE proteins are selected from AvrBs3 or AvrBs4, and said restriction endonucleases are is a genetically modified variant of Pvull.

While Pvull adds an extra element of specificity when combined with TALE proteins, TALE-Pvull chimeric nucleases according to the invention are designed such that only Pvull sites with adjacent zinc finger (ZF) binding sites which constitute the binding sites of TALE proteins are cleaved, meaning that only addressed Pvull sites are cleaved but no off-target Pvull sites. To achieve this, suitable linkers and introduced amino acid substitutions and/or deletions or additions into Pvull that alter activity and increase fidelity are selected, thereby making DNA cleavage by Pvull dependent on the DNA binding by the TALE protein. Therefore, neither the DNA binding domain nor the cleavage domain alone shall be sufficient for allowing functional interaction of the chimeric nuclease with DNA.

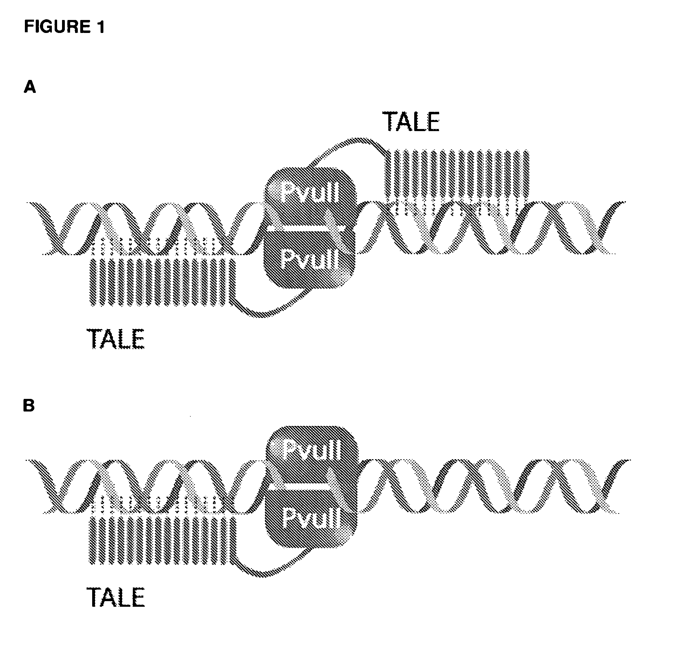

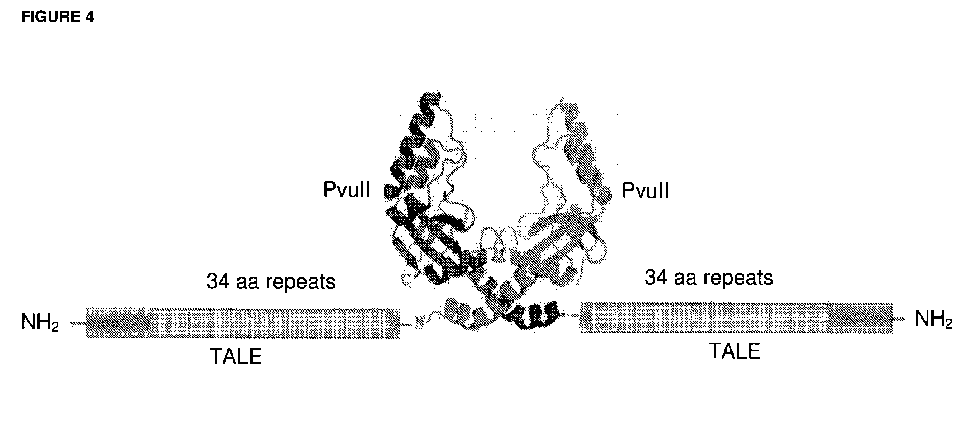

Preferably, in order to be functional, the chimeric nuclease will form a homodimer comprising two chimeric nuclease monomers having the structure of the chimeric nucleases of the invention. The cleavage domains of these monomers (TALE-Pvull) interact with each other and thereby mediate formation of the homodimer. The dimerized chimeric nucleases then recognize a specific bipartite or tripartite DNA binding site comprising one or two recognition site(s) of the DNA binding module and a recognition site of the cleavage module (SEQ ID No. 7). For example, homodimerization is mediated by a cleavage module derived from the restriction endonuclease Pvull (FIG. 1A, FIG. 4; SEQ ID No. 5). Alternatively, the chimeric nucleases which form a dimer comprise DNA binding modules derived from different TALE proteins. For example, one monomer exhibits a DNA binding module derived from TALE protein AvrBs3, and the second monomer exhibits a DNA binding module derived from TALE protein AvrBs4.

Alternatively, in a so-called single chain variant (TALE-scPvull) the cleavage domain comprises two Pvull derived amino acid sequences, each forming a Pvull monomer, which are fused via a linker according to the present invention to a DNA binding domain derived from a TALE protein (FIG. 1 B). According to the invention, the bipartite or tripartite DNA target for the chimeric nucleases comprising a DNA binding domain derived from the TALE protein AvrBs3 or AvrBs4, a linker, and a cleavage domain derived from the restriction endonuclease Pvull is given in the accompanying SEQ ID No. 7.

The present invention also provides a vector comprising the polynucleotide encoding the chimeric nuclease as referred to herein. Said vectors are obtained by conventional polymerase chain reaction (PCR) techniques using appropriate primer sequences and subsequent cloning methods. Said techniques and methods are well-known by skilled persons and belong to the prior art. Suitable cloning vectors for the purpose of the invention comprise plasmids, viral or retroviral vectors, phages, and artificial chromosomes such as bacterial or yeast artificial chromosomes. Preferably, vectors used in the invention comprise selectable markers for propagation and/or selection in a host organism as well as expression control sequences allowing expression of the operatively linked polynucleotide in prokaryotic or eukaryotic host cells. Such vectors are well-known to the skilled worker and particularly include vectors that are able to replicate in microorganisms, thereby ensuring efficient cloning in bacteria, yeasts, and fungi. Preferably, vectors are used for expression of proteins in prokaryotes that comprise constitutive or inducible promoters which govern the protein expression. Classical vectors that are known to the skilled worker are, amongst others, for example pQE30, pASK, pLGM, pGEX, pET, pTrc, pMAL, pUC18, pUC19, pBR322, pACYC184, pLG338, pLG200, M113mp, pKC30, pRep4, pHS1, pHS2, pPLc236, pMBL24, pUR290, pIN-III113-B1, λgt11 or pBdCl, in Streptomyces pIJ101, plJ364, plJ702 or plJ361, in Bacillus pUB110, pC194 or pBD214, in Corynebacterium pSA77 or pAJ667, and series variants thereof. Suitable yeast expression vector comprise for example pYeDesaturasec1, pMFa, pJRY88, pYES2, pAG-1, YEp6, YEp13, or pEMBLYe23. Suitable vectors for expression in insect cells are for example Baculovirus expression vectors. Suitable vectors for expression in eukaryotic cells are for example pBluescript, pCDM8, pcDNA1, pcDNA3, pSPORT1, pcDV1. Most preferably, pQE30 is used as a vector for expression of the chimeric nuclease as referred to herein.

Introduction of these vectors into prokaryotic or eukaryotic host cells is performed by conventional transformation, transfection, conjugation, and transduction techniques that all constitute methods known in the prior art for introduction of foreign nucleic acids such as DNA into a host cell. These methods comprise for example lipofection, electroporation, chemically mediated transfer, particle bombardement, calcium phosphate or calcium chloride coprecipitation, DEAE-dextrane mediated transfection, heat shock, appropriate packaging cell lines.

Manufacturing of chimeric nucleases according to the present invention:
The vectors comprising the polynucleotide encoding the chimeric nucleases as referred to herein according to the present invention are used for manufacturing of the chimeric nucleases by conventional techniques that are known to the skilled worker. These techniques include chemical synthesis or recombinant molecular biology techniques. Preferably, manufacturing of the chimeric nucleases comprises (i) culturing the host cell and (ii) obtaining the chimeric nuclease from said host cell. Preferably, the chimeric nuclease is obtained by conventional purification techniques from a lysate of host cells. Said purification techniques comprise for example affinity chromatography, ion exchange chromatography, size exclusion chromatography, hydrophobic interaction chromatography and/or preparative gel electrophoresis. Details on the testing for the desired enzymatic activity of the chimeric nucleases are described in the examples below.

Use of chimeric nucleases and vectors encoding them for gene targeting
The vectors comprising the polynucleotide encoding the chimeric nucleases as referred to herein according to the present invention are used for highly specific gene targeting efforts. The expressed chimeric nucleases will thereby facilitate the integration of heterologous nucleic acid of interest, for example DNA, such as a transgene, at a predefined site within a target nucleic acid, such as genomic DNA, of the host which preferably is a microorganism, animal, plant, or human. Furthermore, the chimeric nucleases also support homologous recombination events. Thus, the chimeric nucleases will be used for targeted transgenesis or gene insertion or gene knock-out or gene inactivation or homologous replacement or knock-in or gene replacement. Said methods are well-known by the skilled worker. Gene targeting processes and applications for modifying the germ line genetic identity of human beings are not part of the present invention.

The present invention thereby significantly improves the generation of transgenic organisms including transgenic microorganisms, animals plants, or human. Transgenic organisms will be obtained by introduction of
a) the vector comprising the polynucleotide encoding the chimeric nuclease and
b) a nucleic acid of interest to be introduced into the host cell genome
into the host cell by conventional transformation, transfection, conjugation, or transduction techniques that all constitute methods known in the prior art for introduction of heterologous nucleic acids such as DNA into a host cell.

After introduction of said vector and heterologous nucleic acid of interest into the host cell, the chimeric nuclease will be expressed. Subsequently, the chimeric nuclease will be recruited to the bipartite or tripartite genomic DNA recognition site, thereby allowing cleavage of the genomic DNA by the chimeric nuclease and subsequent integration of the heterologous nucleic acid of interest into the genomic DNA at the preferred site.

Moreover, the present invention is used as a rare cutting endonuclease supporting conventional cloning efforts known in the prior art.

### Examples

The examples below illustrate the present invention.

### Example 1: Cloning of TALE-Pvull and TALE-scPvull chimeric nucleases

A chimeric nuclease according to the present invention comprising a DNA binding domain derived from TALE protein AvrBs3 or AvrBs4, respectively, or variants thereof, a linker, and a cleavage domain derived from the restriction endonuclease Pvull (Pvull wildtype or variants thereof) is engineered by the following steps. First, the coding sequences or parts thereof for the TALE proteins (AvrBs3 or AvrBs4, respectively), for an amino acid linker (as for example consisting of the amino acids SSVIPNRGVTKQLVK), and for Pvull or single chain Pvull (scPvull) or parts thereof are amplified by conventional PCR using appropriate primer sequences hybridizing to DNA encoding Pvull wildtype or variants thereof. For amplification of sequences encoding TALE proteins AvrBs3 or AvrBs4, respectively, primers according to SEQ ID No. 8 and SEQ ID No. 9 are used for synthesis of PCR fragments encoding TALE-Pvull (2423 bp) harbouring a long variant of TALE or primers according to SEQ ID No. 8 and SEQ ID No. 10 for synthesis of PCR fragments encoding TALE-scPvull (2319 bp) harbouring a short variant of TALE. For both variants of TALE, plasmid pENTR-D-avrBs3 or pENTR-D-avrBs4, respectively, is used as template (plasmids described in: Morbitzer, R., Elsaesser, J., Hausner, J. & Lahaye, T. (2011). Assembly of custom TALE-type DNA binding domains by modular cloning. Nucleic Acids Res. 39, 5790-5799; Mussolino, C., Morbitzer, R., Lutge, F., Dannemann, N., Lahaye, T. & Cathomen, T. (2011). A novel TALE nuclease scaffold enables high genome editing activity in combination with low toxicity. Nucleic Acids Res.). Furthermore, primer sequences designed according to standard procedures encoding a Strep-tag (Strepll; WSHPQFEK) for purification efforts or a linker amino acid sequence are used for generation of PCR fragments encoding a Strep-tag or a linker, respectively, and appropriate restriction sites for ligation with TALE and Pvull PCR fragments into vector pQE30 (Qiagen). Subsequently, the generated PCR fragments are inserted into plasmid pQE30 encoding a C-terminal 6His-tag, by conventional techniques, as for example ligation at appropriate temperatures. The generated plasmid is then transformed into *Escherichia coli* and propagated in conventional culture medium at appropriate conditions. Transformed cells are harvested and lysed, and generated plasmid is prepared by conventional methods. Alternatively, mutations encoding amino acid substitutions, as for example T46G in Pvull, are introduced into the generated vectors via PCR-based directed mutagenesis. The generated plasmids encode a) a chimeric nuclease (TALE-Pvull; Seq ID No. 5; homodimer FIG. 1A) harbouring 1117 amino acid residues, or b) a chimeric nuclease, also named single chain Pvull variant (TALE-scPvull; Seq ID No. 6, FIG. 1 B) herein, harbouring 992 amino acid residues, respectively, while the chimeric nuclease of a) exhibits the following domain architecture N-Strepll-TALE/AvrBs3(aa153-914)-Linker-Pvull(aa2-157;T46G)-Pvull(aa2-157;T46G)-6His-C, and the chimeric nuclease of b) exhibits the following domain architecture N-Strepll-TALE/AvrBs4(aa153-949)-Linker-Pvull(aa2-157;T46G)-6His-C (N: N-terminus; C: C-terminus; aa: amino acid residues, numbered according to wildtype Pvull; T46G: amino acid substitutions).

### Example 2: Purification of chimeric nucleases

For the protein expression of chimeric nucleases according to the present invention a 25 ml LB medium pre-culture (containing 75 µg/ml Ampicillin) is inoculated and grown overnight at 37°C. Further, 5 ml of this pre-culture are added to a 500 ml culture (containing 75 g/ml Ampicillin) and the cells are grown until an optical density of 0.6-0.8 at 600 nm (OD₆₀₀). The protein expression is induced by adding 5 ml of 0.1 M Isopropyl β-D-1-thiogalactopyranoside (1 mM end concentration) to the 500 ml cell culture. Finally, the cell culture is incubated overnight at 18 °C. The cells are harvested by centrifugation at 5000 g for 15 minutes and the pellet is resuspended in 40 ml STE buffer (10 mM Tris/HCI pH 8, 100 mM NaCl, 0.1 mM EDTA). Following on this, the culture is centrifuged a second time at 5,000 g for 15 minutes Afterwards, the supernatant is discharged and the pellet is stored at - 20 °C for further processing.

Pellets are first resuspended in 20 ml high-salt buffer (20 mM KPi pH 7.8, 1 M KCI, 0.5 mM EDTA, 1 mM DTT, 0.01 % Lubrol, 1 mM phenylmethylsulfonyl fluoride) and cell lysis is performed on ice by sonication (15 sec pulses for 6 minutes at 50% output) of the cells using a *Sonifier 250, Branson*. After sonication, the lysate is centrifuged at 48,000 g for 30 minutes at 4 °C and the supernatant is filtered using a 0.45µm cellulose acetate filter.

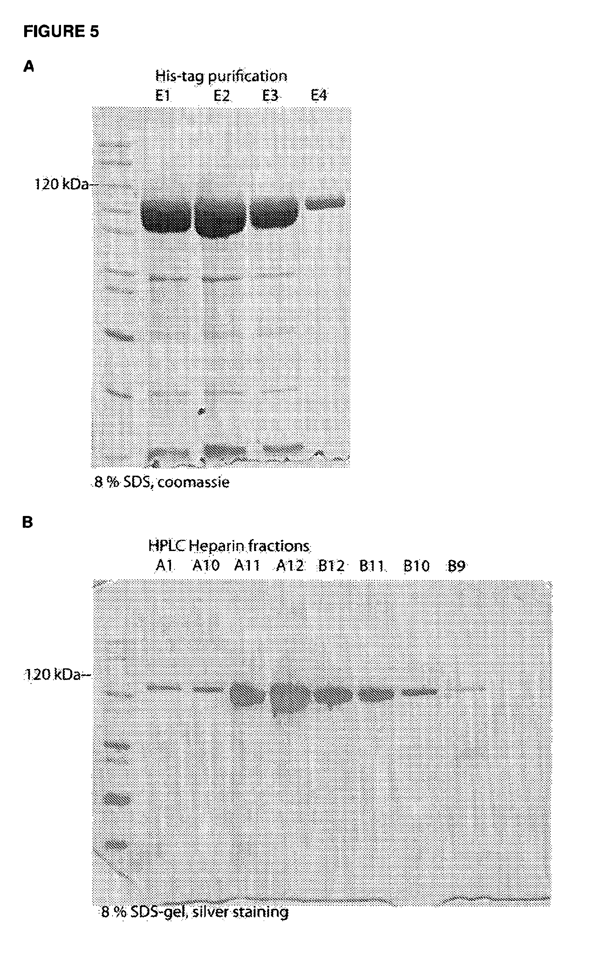

The protein purification is performed via immobilized metal assisted chromatography using 1 ml/l cell culture Ni-NTA (Qiagen) as a chelator (His-tag purification). Therefore the column is equilibrated with two column volumes high salt buffer and the lysate is loaded onto the column. The following steps are all done at 4 °C to avoid protein denaturation. The column is washed twice with 2 ml high salt buffer and 2 ml low salt buffer (20 mM KPi pH 7.2, 300 mM KCI, 0.5 mM EDTA, 1 mM DTT, 0.01 % Lubrol, 1 mM phenylmethylsulfonyl fluoride). Finally the protein is eluted with 4x 500 µl elution buffer (20 mM KPi pH 7.8, 500 mM KCI, 0.5 mM EDTA, 1 mM DTT, 0.01% Lubrol, 1 mM phenylmethylsulfonyl fluoride, 250 mM imidazole). From each eluate 15 µl samples are taken and mixed with 3 µl 5x Laemmli loading buffer for further SDS PAGE analyses (FIG. 5A).

Fusion protein containing fractions are pooled and further purified via Heparin chromatography. Therefore the pooled fractions are loaded onto a 5ml HiTrap™ Heparin HP Column, washed with one column volume low salt buffer and eluted using a continuous gradient (20 ml) from low salt buffer to elution buffer (20 mM KPi pH 7.8, 1000 mM KCI, 0.5 mM EDTA, 1 mM DTT, 0.01% Lubrol). The protein elutes corresponding to a buffer with 550 mM KCI. From each eluate 10 µl samples are taken and mixed with 2 µl 5x Laemmli loading buffer for further SDS PAGE analyses (FIG. 5B).

Finally, the chosen protein fractions are pooled and dialysed (Snakeskin®dialysis tubing, 10 k MWCO) overnight at 4 °C using 30 mM KPi pH 7.8, 550 mM KCI, 1 mM EDTA, 1 mM DTT, 60 % glycerol. After dialysis the protein concentration is determined by conventional methods.

### Example 3: Functional activity assay of TALE-scPvull chimeric nuclease according to examples 1 and 2 with an addressed or a non-addressed DNA substrate

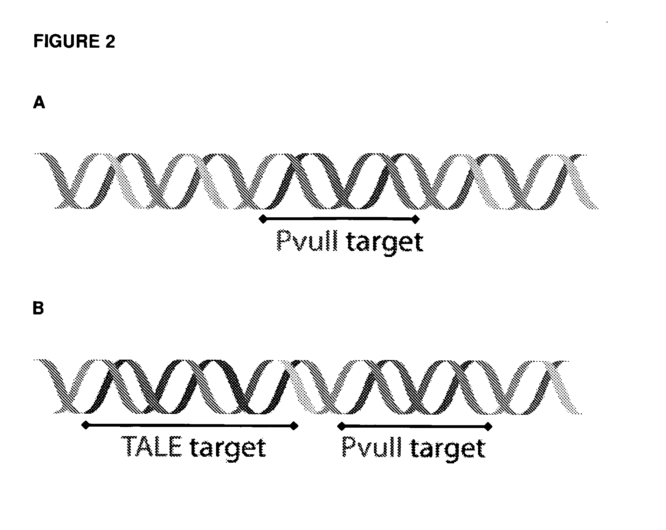

For DNA cleavage assays, the different DNA target sequences containing one Pvull recognition site (= non-addressed site; FIG. 2A) or one Pvull recognition site plus TALE recognition site(s) (= addressed site; FIG. 2B, SEQ ID No. 7), respectively, were introduced into plasmid pAT153 (= DNA substrate). The DNA cleavage activity assays are carried out with a 1:1 ratio of chimeric nuclease TALE-scPvull (8 nM) and DNA substrate (8 nM DNA, 300 ng). The reaction mixture is incubated at 37 °C for 1, 3, 5, 10, 20, 30, or 60 min, respectively, in buffer containing 20 mM Tris-acetate, 50 mM K-acetate, 2 mM Mg-acetate, pH 7.9. Reaction products are separated on 1% agarose gels by electrophoresis, stained with ethidium bromide, and visualized by the BioDocAnalyze system.

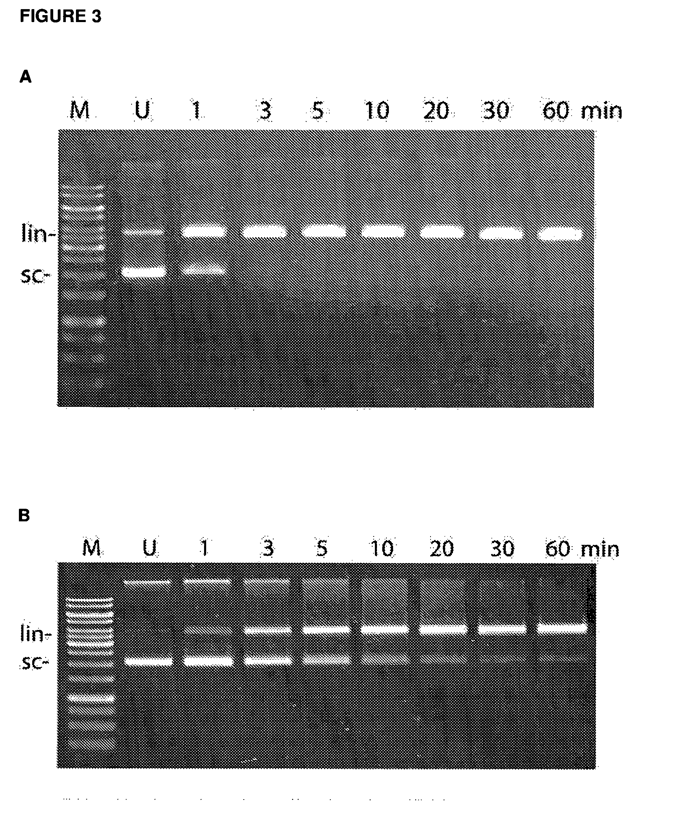

The chimeric nuclease TALE-scPvull prefers by a factor of 10 the addressed (TALE recognition site plus Pvull recognition site; bipartite; FIG. 2B, FIG. 4) over the unaddressed (only Pvull recognition site; Fig. 2A) DNA recognition site (FIG. 3).

### Description of the drawings

**Figure 1.** Figure 1A shows a homodimer consisting of two TALE-Pvull chimeric nuclease monomers while binding to a tripartite DNA target site comprising one Pvull recognition site and two TALE protein recognition sites. Figure 2B shows a TALE-scPvull chimeric nuclease while binding to a bipartite DNA target site comprising one Pvull recognition site and one TALE protein recognition site, wherein two fused Pvull polypeptides within the cleavage domain form a homodimer.
**Figure 2.** Figure 2A illustrates a single Pvull recognition site (Pvull target) within the DNA which is not addressed by the chimeric nucleases as referred to in the present invention. Figure 2B shows a bipartite DNA recognition site comprising a Pvull recognition site (Pvull target) and a TALE protein recognition site (TALE target) which is addressed by the chimeric nucleases as referred to in the present invention.
**Figure 3.** Figure 3A shows a DNA cleavage assay, wherein purified TALE-scPvull chimeric nuclease was incubated for 1, 3, 5, 10, 20, 30, or 60 minutes, respectively, at an equimolar amount of 8 nM with DNA encoding the addressed bipartite DNA target site (according to FIG. 2B, SEQ ID No. 7). Since the DNA target site is embedded in a plasmid (sc), DNA binding and subsequent cleavage of the DNA target site results in al linearized plasmid (lin). Figure 3B shows a DNA cleavage assay according to Figure 3A but wherein the DNA encodes the non-addressed only Pvull DNA target site (according to FIG. 2A). (M = marker DNA; U = only plasmid without TALE-scPvull).
**Figure 4**. Figure 4 is a cartoon of two TALE-Pvull chimeric nuclease monomers forming a homodimer, while the TALE parts of the monomers exhibit 34 amino acid (aa) repeats each. The TALE parts of the chimeric nuclease monomers are each linked to the mainly alpha-helical structure of Pvull within the cleavage domain of the chimeric nuclease. Homodimerization occurs via interaction of the Pvull parts of the chimeric nuclease monomers.
**Figure 5**. Figure 5A shows the elution fractions E1 to E4 of chimeric nuclease purified using Ni-NTA affinity chromatography. The fractions are loaded onto a 8% SDS gel, separated by electrophoresis, and stained with coomassie. Figure 5B shows the elution fractions A1, A10, A11, A12, B12, B11, B10, and B9 of chimeric nuclease purified in a second step following Ni-NTA purification via Heparin chromatography (HPLC). The fractions are loaded onto a 8% SDS gel, separated by electrophoresis, and stained with silver.

The following sequences referred to herein are shown in the accompanying sequence listing:
**SEQ ID No. 1:** TALE AvrBs3
**SEQ ID No. 2:** TALE AvrBs4
**SEQ ID No. 3:** Pvull
**SEQ ID No. 4:** Linker SSVIPNRGVTKQLVK
**SEQ ID No. 5:** TALE-Pvull
**SEQ ID No. 6:** TALE-scPvull
**SEQ ID No. 7:** bipartite/tripartite DNA target site
**SEQ ID No. 8:** TALE forward
**SEQ ID No. 9:** TALE reverse-1
**SEQ ID No. 10:** TALE reverse-2

## Claims

1. A chimeric nuclease which comprises the following components:
- a module comprising at least a DNA binding domain from TALE protein AvrBs3 (SEQ ID NO: 1) and/or a DNA binding domain from TALE protein AvrBs4 (SEQ ID NO: 2),
- a linker composed of at least five amino acid residues, and
- a module comprising at least a cleavage domain of a type IIP restriction endonuclease Pvull either having an amino acid sequence according to SEQ ID NO: 3 or having an amino acid sequence according to SEQ ID NO: 3 with one of following modifications: amino acid substitutions D5K, L12E, P14G, H15D, Q17E, K25D, K25E, N62D, K78E, H83A, Y94F, K155E, or K174E, respectively, or combinations thereof, or amino acid deletions delta 1-15 or delta 1-25 and
wherein said chimeric nuclease functionally interacts only with DNA that comprises both a DNA recognition site for said DNA binding domain from a TALE protein and a DNA recognition site for said cleavage domain from a restriction endonuclease, and
wherein said cleavage domain cleaves the DNA upon binding of the chimeric nuclease to the DNA.

2. The chimeric nuclease of claim 1, wherein said DNA binding module exhibits reduced or no catalytic activity.

3. The chimeric nuclease of claims 1 or 2, wherein said linker essentially consists of 5 to 20 amino acids.

4. The chimeric nuclease of any one of claims 1 to 3, wherein said linker has an amino acid sequence as shown in SEQ ID No. 4 (SSVIPNRGVTKQLVK).

5. The chimeric nuclease according to claim 4, wherein the chimeric nuclease recognizes a bipartite or tripartite DNA target site comprising one Pvull recognition site and one or two TALE protein recognition site(s) according to SEQ ID No. 7.

6. A vector encoding the chimeric nuclease of any one of claims 1 to 4.

7. A method for manufacturing a chimeric nuclease according to any one of claims 1 to 5, wherein
manufacturing comprises the following steps:
(i) Transformation of a vector according to claim 6 encoding a chimeric nuclease into a microorganism,
(ii) cultivation of the transformed microorganism while inducing expression of the chimeric nuclease,
(iii) harvesting and lysis of the microorganism,
(iv) purification of the chimeric nuclease from lysed microorganism cells.

8. The chimeric nuclease according to any one of claims 1 to 5 for use in gene targeting of a microorganism, or an animal tissue or isolated cell thereof, or a plant, or a plant part or isolated cell thereof, or a human wherein processes for modifying the germ line genetic identity of human beings are excluded.

9. A non-human organism comprising the chimeric nuclease of any one of claims 1-5 or the vector encoding the chimeric nuclease of claim 6, wherein the organism is a microorganism, or an isolated cell of an animal tissue, or a plant, or a plant part or isolated cell thereof.

## Patentansprüche

1. Chimäre Nuklease, welche die folgenden Komponenten umfasst:
- ein Modul umfassend wenigstens eine DNA-bindende Domäne des TALE-Proteins AvrBs3 (SEQ ID NO: 1) und/oder eine DNA-bindende Domäne des TALE-Proteins AvrBs4 (SEQ ID NO: 2),
- einen Linker zusammengesetzt aus wenigstens fünf Aminosäureresten, und
- ein Modul umfassend wenigstens eine Spaltdomäne einer Restriktionsendonuklease Pvull vom IIP-Typ entweder mit einer Aminosäuresequenz gemäß SEQ ID NO: 3 oder mit einer Aminosäuresequenz gemäß SEQ ID NO: 3 mit einer der folgenden Modifikationen: Aminosäuresubstitutionen D5K, L12E, P14G, H15D, Q17E, K25D, K25E, N62D, K78E, H83A, Y94F, K155E bzw. K174E oder Kombinationen davon oder Aminosäuredeletionen delta 1-15 oder delta 1-25 und
wobei die chimäre Nuklease funktional nur mit DNA wechselwirkt, die sowohl eine DNA-Erkennungsstelle für die DNA-Bindungsdomäne eines TALE-Proteins als auch eine DNA-Erkennungsstelle für die Spaltdomäne einer Restriktionsendonuklease umfasst, und
wobei die Spaltdomäne die DNA nach dem Binden der chimären Nuklease an die DNA spaltet.

2. Chimäre Nuklease nach Anspruch 1, wobei das DNA-bindende Modul eine verringerte oder keine katalytische Aktivität aufweist.

3. Chimäre Nuklease nach Anspruch 1 oder 2, wobei der Linker im Wesentlichen aus 5 bis 20 Aminosäuren besteht.

4. Chimäre Nuklease nach einem der Ansprüche 1 bis 3, wobei der Linker eine Aminosäuresequenz hat, wie sie in SEQ ID NO: 4 gezeigt ist (SSVIPNRGVTKQLVK).

5. Chimäre Nuklease nach Anspruch 4, wobei die chimäre Nuklease einen zweiteiligen oder dreiteiligen DNA-Zielort erkennt, welcher eine Pvull-Erkennungsstelle und ein oder zwei TALE-Protein-Erkennungsstellen gemäß SEQ ID NO: 7 umfasst.

6. Vektor kodierend die chimäre Nuklease nach einem der Ansprüche 1 bis 4.

7. Verfahren zum Herstellen einer chimären Nuklease nach einem der Ansprüche 1 bis 5, wobei
Herstellen die folgenden Schritte umfasst:
(i) Transformieren eines Vektors nach Anspruch 6, welcher eine chimäre Nuklease kodiert, in einen Mikroorganismus,
(ii) Kultivieren des transformierten Mikroorganismus während eine Expression der chimären Nuklease induziert wird,
(iii) Ernten und Lysieren des Mikroorganismus,
(iv) Aufreinigen der chimären Nuklease aus lysierten Mikroorganismuszellen.

8. Chimäre Nuklease nach einem der Ansprüche 1 bis 5 zur Verwendung im Gen-Targeting eines Mikroorganismus oder eines tierischen Gewebes oder einer isolierten Zelle davon oder einer Pflanze oder eines Pflanzenteils oder einer isolierten Zelle davon oder eines Menschen, wobei Verfahren zum Modifizieren der genetischen Identität der Keimbahn von menschlichen Wesen ausgeschlossen sind.

9. Nichthumaner Organismus umfassend die chimäre Nuklease nach einem der Ansprüche 1 bis 5 oder den Vektor kodierend die chimäre Nuklease nach Anspruch 6, wobei der Organismus ein Mikroorganismus oder eine isolierte Zelle aus einem tierischen Gewebe oder eine Pflanze oder ein Pflanzenteil oder eine isolierte Zelle davon ist.

## Revendications

1. Nucléase chimérique qui comprend les composants suivants :
- un module comprenant au moins un domaine de liaison d'ADN provenant de la protéine TALE AvrBs3 (SEQ ID n° 1) et/ou un domaine de liaison d'ADN provenant de la protéine TALE AvrBs4 (SEQ ID n° 2),
- un lieur composé d'au moins cinq résidus d'acide aminé, et
- un module comprenant au moins un domaine de clivage d'une endonucléase de restriction PvuII de type IIP soit ayant une séquence d'acides aminés selon SEQ ID n° 3 soit ayant une séquence d'acides aminés selon SEQ ID n° 3 avec une des modifications suivantes : substitutions d'acide aminé D5K, L12E, P14G, H15D, Q17E, K25D, K25E, N62D, K78E, H83A, Y94F, K155E, ou K174E, respectivement, ou des combinaisons de celles-ci, ou délétions d'acides aminés delta 1-15 ou delta 1-25, et
dans laquelle ladite nucléase chimérique interagit fonctionnellement seulement avec un ADN qui comprend tant un site de reconnaissance d'ADN pour ledit domaine de liaison d'ADN provenant d'une protéine TALE qu'un site de reconnaissance d'ADN pour ledit domaine de clivage provenant d'une endonucléase de restriction, et
dans laquelle le domaine de clivage fend l'ADN lors de la liaison de la nucléase chimérique à l'ADN.

2. Nucléase chimérique selon la revendication 1, dans laquelle ledit module de liaison d'ADN présente une activité catalytique réduite ou nulle.

3. Nucléase chimérique selon les revendications 1 ou 2, dans laquelle ledit lieur consiste sensiblement en 5 à 20 acides aminés.

4. Nucléase chimérique selon n'importe laquelle des revendications 1 à 3, dans laquelle ledit lieur a une séquence d'acides aminés comme le montre SEQ ID n° 4 (SSVIPNRGVTKQLVK).

5. Nucléase chimérique selon la revendication 4, dans laquelle la nucléase chimérique reconnaît un site de cible d'ADN bipartite ou tripartite comprenant un site de reconnaissance de PvuII et un ou deux site (s) de reconnaissance de protéine TALE selon SEQ ID n° 7.

6. Vecteur codant la nucléase chimérique selon n'importe laquelle des revendications 1 à 4.

7. Procédé pour fabriquer une nucléase chimérique selon n'importe laquelle des revendications 1 à 5, dans lequel
la fabrication comprend les étapes suivantes :
(i) Transformation d'un vecteur selon la revendication 6 codant une nucléase chimérique dans un micro-organisme,
(ii) culture du micro-organisme transformé tout en induisant expression de la nucléase chimérique,
(iii) récolte et lyse du micro-organisme,
(iv) purification de la nucléase chimérique à partir de cellules de micro-organisme lysées.

8. Nucléase chimérique selon n'importe laquelle des revendications 1 à 5 à utiliser dans le ciblage génétique d'un micro-organisme, ou d'un tissu animal ou d'une cellule isolée de celui-ci, ou d'une plante, ou d'une partie de plante ou d'une cellule isolée de celle-ci, ou d'un humain dans lequel des processus pour modifier l'identité génétique de lignée germinale d'êtres humains sont exclus.

9. Organisme non humain comprenant la nucléase chimérique selon n'importe laquelle des revendications 1 à 5 ou le vecteur codant la nucléase chimérique selon la revendication 6, dans lequel l'organisme est un micro-organisme, ou une cellule isolée d'un tissu animal, ou une plante, ou une partie de plante ou une cellule isolée de celle-ci.
